# EUROPEAN PATENT APPLICATION

(11) **EP 1 845 154 A1**
(43) Date of publication of application: **17.10.2007**
(21) Application number: 07006923.2
(22) Date of filing: 03.04.2007
(51) Int. Cl.: C12N 5/06

(54) **Multipotent stem cells derived from placenta tissue and cellular therapeutic agents comprising the same**

(30) Priority: 12.04.2006 KR 20060033325; 23.01.2007 KR 20070007139
(71) Applicant: RNL Bio Co., Ltd., Yangjae-dong, Seocho-gu Seoul 137-130 (KR)
(72) Inventor: Ra, Jeong Chan, Suwon-si, Gyeonggi-do, 440-300 (KR); Kim, Bong Hui, Yongin-si, Gyeonggi-do, 448-172 (KR); Lee, Hang Young, Cheongju-si, Chungbuk, 361-280 (KR); Woo, Sang Kyu, Gyeonggi-do, 431-080 (KR); Park, Hanna, Jeju-si, Jeju-do, 690-817 (KR); Kim, Hyoeun, Seoul, 152-770 (KR)
(74) Representative: Hiebl, Inge Elisabeth

(57) **Abstract**

The present invention relates to placenta tissue-derived multipotent stem cells and cell therapeutic agents containing the same. More specifically, to a method for producing placenta stem cells having the following characteristics, the method comprising culturing amnion, chorion, decidua or placenta tissue in a medium containing collagenase and bFGF and collecting the cultured cells: (a) showing a positive immunological response to CD29, CD44, CD73, CD90 and CD105, and showing a negative immunological response to CD31, CD34, CD45 and HLA-DR; (b) showing a positive immunological response to Oct4 and SSEA4; (c) growing attached to plastic, showing a round-shaped or spindle-shaped morphology, and forming spheres in an SFM medium so as to be able to be maintained in an undifferentiated state for a long period of time; and (d) having the ability to differentiate into mesoderm-, endoderm- and ectoderm-derived cells. Also the present invention relates to placenta stem cells obtained using the production method. The inventive multipotent stem cells have the ability to differentiate into muscle cells, vascular endothelial cells, osteogenic cells, nerve cells, satellite cells, fat cells, cartilage-forming cells, osteogenic cells, or insuline-secreting pancreatic β-cells, and thus are effective for the treatment of muscular diseases, osteoporosis, osteoarthritis, nervous diseases, diabetes and the like, and are useful for the formation of breast tissue.

## Description

### BACKGROUND OF THE INVENTION

### Field of the Invention

The present invention relates to placenta tissue-derived multipotent stem cells and cell therapeutic agents containing the same, and more particularly, to placenta stem cells having the following characteristics: (a) showing a positive immunological response to CD29, CD44, CD73, CD90 and CD105, and showing a negative immunological response to CD31, CD34, CD45 and HLA-DR; (b) showing a positive immunological response to Oct4 and SSEA4; (c) growing attached to plastic, showing a round-shaped or spindle-shaped morphology, and forming spheres in an SFM medium so as to be able to be maintained in an undifferentiated state for a long period of time; and (d) having the ability to differentiate into mesoderm-, endoderm- and ectoderm-derived cells.

### Background of the Related Art

Biotechnology for the 21^{st} century presents the possibility of new solutions to the food, environment and health problems, with the ultimate object of promoting human prosperity. In recent years, the technology of using stem cells has been considered as a new way to treat incurable diseases. Formerly, organ transplantation, gene therapy, etc., were presented for the treatment of incurable human diseases, but their efficient use has not been made due to immune rejection, short supply of organs, an insufficient development of vectors, and an insufficient knowledge of disease genes.

For this reason, with increasing interests in stem cell studies, it has been recognized that totipotent stem cells having the ability to form all the organs by proliferation and differentiation can not only treat most of diseases but also fundamentally heal organ injuries. Also, many scientists have suggested the applicability of stem cells for the regeneration of all the organs and the treatment of incurable diseases, including Parkinson's disease, various cancers, diabetes and spinal damages.

Stem cells refer to cells having not only self-replicaiton ability but also the ability to differentiate into at least two cells, and can be divided into totipotent stem cells, pluripotent stem cells, and multipotent stem cells.

Totipotent stem cells are cells having totipotent properties capable of developing into one perfect individual, and these properties are possessed by cells up to the 8-cell stage after the fertilization of an oocyte and a sperm. When these cells are isolated and transplanted into the uterus, they can develop into one perfect individual.

Pluripotent stem cells, which are cells capable of developing into various cells and tissues derived from the ectodermal, mesodermal and endodermal layers, are derived from an inner cell mass located inside of blastocysts generated 4-5 days after fertilization. These cells are called "embryonic stem cells" and can differentiate into various other tissue cells but not form new living organisms.

Multipotent stem cells, which are stem cells capable of differentiating into only cells specific to tissues and organs containing these cells, are involved not only in the growth and development of various tissues and organs in the fetal, neonatal and adult periods but also in the maintenance of homeostasis of adult tissue and the function of inducing regeneration upon tissue damage. Tissue-specific multipotent cells are collectively called "adult stem cells".

Adult stem cells are obtained by collecting cells from various human organs and developing the cells into stem cells and are characterized in that they differentiate into only specific tissues. However, recently, experiments for differentiating adult stem cells into various tissues, including liver cells, have been dramatically successful.

The multipotent stem cells were first isolated from adult bone marrow (Jiang et al., Nature, 418:41, 2002), and then also found in other various adult tissues (Verfaillie, Trends Cell Biol., 12:502, 2002). In other words, although the bone marrow is the most widely known source of stem cells, the multipotent stem cells were also found in the skin, blood vessels, muscles and brains (Tomas et al., Nat. Cell Biol., 3:778, 2001; Sampaolesi et al., Science, 301:487, 2003; Jiang et al., Exp. Hematol., 30:896, 2002). However, stem cells in adult tissues, such as the bone marrow, are very rarely present and such cells are difficult to culture without inducing differentiation, and thus difficult to culture in the absence of specifically screened media. Namely, it is very difficult to maintain the isolated stem cells *in vitro.*

Recently, adipose tissue was found to be a new source of multipotent stem cells (Cousin et al., BBRC., 301:1016, 2003; Miranville et al., Circulation, 110:349, 2004; Gronthos et al., J. Cell Physiol., 189:54, 2001; Seo et al., BBRC., 328:258, 2005). Namely, it was reported that a group of undifferentiated cells is included in human adipose tissue obtained by liposuction and has the ability to differentiate into fat cells, osteogenic cells, myoblasts and chondroblasts (Zuk et al., Tissue Eng., 7:211, 2001; Rodriguez et al., BBRC., 315:255, 2004). This adipose tissue has an advantage in that it can be extracted in large amounts, and thus, it receives attention as a new source of stem cells, which overcomes the existing shortcomings. Also, recent studies using animal model experiments indicate that adipose tissue-derived cells have the abilities to regenerate muscles and to stimulate the differentiation of nerve blood vessels. Thus, these adipose tissue-derived cells receive attention as a new source of stem cells.

Human stem cells are totipotential or pluripotential precursor cells capable of generating a variety of mature human cell lineages. This ability serves as the basis for the cellular differentiation and specialization necessary for organ and tissue development. Recent success at transplanting such stem cells have provided new clinical tools to reconstitute and/or supplement the bone marrow after myeloablation due to disease, exposure to toxic chemical and/or radiation. Further evidence exists, which demonstrates that stem cells can be employed to repopulate many if not all of tissues and restore physiologic and anatomic functionality. Also, the application of stem cells in tissue engineering, gene therapy delivery and cell therapeutic agents is also advancing rapidly. Accordingly, in the art to which the present invention pertains, as interest in stem cells is increasing, the development and production of stem cells from various tissues are needed.

Accordingly, the present inventors have made many efforts to produce stem cells from various tissues and, as a result, found that when placenta stem cells are produced from placenta tissue, they can be efficiently produced in terms of immunology compared to the existing method of producing stem cells from fat tissue, thereby completing the present invention.

### SUMMARY OF THE INVENTION

Accordingly, it is an object of the present invention to provide placenta-derived multipotent stem cells and a production method thereof.

Another object of the present invention is to provide a method for differentiating said placenta-derived multipotent stem cells into muscle cells, nerve cells, osteogenic cells, fat tissue cells, cartilage cells and pancreatic β-cells.

Still another object of the present invention is to provide cellular therapeutic agents for treating osteoarthritis, osteoporosis and diabetes, and cellular therapeutic agents for forming breast tissue, the cellular therapeutic cells containing said placenta stem cells or cells differentiated therefrom.

To achieve the above objects, in one aspect, the present invention provides a method for producing placenta stem cells having the following characteristics, the method comprising culturing finely cut amnion, chorion, decidua or placenta tissue in a medium containing collagenase and bFGF and collecting the cultured cells: (a) showing a positive immunological response to CD29, CD44, CD 54, CD73, CD90 and CD105, and showing a negative immunological response to CD31, CD34, CD45 and HLA-DR; (b) showing a positive immunological response to Oct4 and SSEA4; (c) growing attached to plastic, showing a round-shaped or spindle-shaped morphology, and forming spheres in an SFM medium so as to be able to be maintained in an undifferentiated state for a long period of time; and (d) having the ability to differentiate into mesoderm-, endoderm- and ectoderm-derived cells.

In another aspect, the present invention provides a method for differentiating said placenta stem cells into muscle cells, nerve cells, osteogenic cells, fat tissue cells, cartilage cells and pancreatic β-cells.

In still another aspect, the present invention provides cellular therapeutic agents containing said placenta stem cells or cells differentiated therefrom, a s active ingredients.

The above and other objects, features and embodiments of the present invention will be more clearly understood from the following detailed description and the accompanying claims.

### BRIEF DESCRIPTION OF THE DRAWINGS

FIG. 1 is microscopic photographs showing the morphology of placenta-derived mesenchymal stem cell (MSC).

FIG. 2 is a graphic diagram showing cumulative population doubling level (CPDL) according to passage number of decidua-derived stem cells.

FIG. 3 is a graphic diagram showing the cumulative population doubling level (CPDL) according to passage number of amnion-derived stem cells.

FIG. 4A is a photograph showing that the first-passage amnion cells cultured in a SFM medium for 3 days formed spheres, and FIG. 4B is a photograph showing that the first-passage decidua cells cultured in a SFM medium for 7 days formed spheres.

FIG. 5 shows the results obtained by analyzing the surface antigens of decidua-derived MSC using a fluorescence activated cell sorter (FACS).

FIG. 6 shows the results obtained by analyzing the surface antigens of amnion-derived MSC using a fluorescence activated cell sorter (FACS).

FIG. 7 is a photograph showing the results of immunohistochemical analysis conduced using specific antibodies [A: OCT4; B: SSEA4; C: CD44, CD54 and control].

FIG. 8 shows the results of RT-PCR for OCT4 [lane 1: marker; lane 2: RT-reaction control; lane 3: amnion stem cells; lane 4: decidua stem cells; and 5: PCR-reaction control].

FIG. 9 is a microscopic photograph showing the results of immunohistochemical analysis (αMyosin-FITC) for amnion-derived stem cells (A) and decidua-derived stem cells (B) induced for 10 days in MM-3160 media (for muscle cell differentiation) in myogenesis.

FIG. 10 is a microscopic photograph showing the results of immunohistochemical analysis (αGFAP-FITC) for amnion-derived stem cells (A) and decidua-derived stem cells (B) induced for 10 days in NM-3229 media (for nerve cell differentiation) in neurogensis.

FIG. 11 is a photograph showing the results of Alizarin red S staining for amnion-derived stem cells (A) and decidua-derived stem cells (C) in osteogenesis [A: control; B: amnion-derived stem cells in osteogenesis; and C: decidua-derived stem cells in osteogenesis].

FIG. 12 is a photograph showing the results of Oil red O staining for amnion-derived stem cells (A) and decidua-derived stem cells (C) in adipogenesis.

### DETAILED DESCRIPTION OF THE INVENTION, AND PREFERRED EMBODIMENT THEREOF

### 1. Definition of terms

As used herein, the term "placenta stem cells" refers to cells that are not derived from the inner cell mass of blastocysts. Stem cells that can be obtained from the placenta include placenta stem cells, pluripotent cells, multipotent cells and progenitor cells.

As used herein, the term "multipotent cells" refers to cells that have the capacity to develop into any subset of approximately 260 cell types in the mammalian body. Unlike pluripotent cells, multipotent cells do not have the capacity to form all of the cell types.

As used herein, the term "progenitor cells" refers to cells that are committed to differentiate into a specific type of cell or to form a specific type of tissue.

As used herein, the term "stem cells" refers to master cells that can reproduce indefinitely to form the specialized cells of tissues and organs. Stem cells are developmental pluripotent or multipotent cells. Stem cells can divide to produce two daughter stem cells, or one daughter stem cells and one progenitor ("transit") cells, which then proliferates into fully differentiated and mature cells in tissue.

As used herein, the term "differentiation" refers to a phenomenon in which the structure or function of cells is specialized during the division, proliferation and growth thereof, that is, the feature or function of cell or tissue of an organism changes in order to perform work given to the cell or tissue. Generally, it refers to a phenomenon in which a relatively simple system is divided into two or more qualitatively different partial systems. For example, it means that a qualitative difference between the parts of any biological system, which have been identical to each other at the first, occurs, for example, a distinction, such as a head or a body, between egg parts, which have been qualitatively identical to each other at the first in ontogenic development, occurs, or a distinction, such as a muscle cell or a nerve cell, between cells, occurs, or the biological system is divided into qualitatively distinguishable parts or partial systems as a result thereof.

As used herein, the term "cell therapeutic agent" refers to a drug used for the purpose of treatment, diagnosis and prevention, which contains a cell or tissue prepared through isolation from man, culture and specific operation (as provided by the US FDA). Specifically, it refers to a drug used for the purpose of treatment, diagnosis and prevention through a series of behaviors of *in vitro* multiplying and sorting living autologous, allogenic and xenogenic cells or changing the biological characteristics of cells by other means for the purpose of recovering the functions of cells and tissues. Cell therapeutic agents are broadly divided, according to the differentiation level of cells, into somatic cell therapeutic agents and stem cell therapeutic agents, and the present invention relates to the stem cell therapeutic agents.

### 2. Isolation and purification of placenta stem cells

The placenta is formed for an embryo during pregnancy and is in the shape of a disk having a weight of about 500 g, a diameter of about 15-20 cm and a thickness of 2-3 cm. One side of the placenta is in contact with the mother body, and the other side is in contact with an embryo. The space in the placenta contains the mother's blood for supplying nutrients to an embryo. The placenta consists of three layers: amnion, chorion and decidua. The amnion is a thin clear membrane surrounding an embryo and contains amniotic fluid, and the stem cells of an embryo are present in the amnion. The decidua is a membrane formed as a result of a process in which the epithelial cells of the uterus are modified so that a fertilized egg becomes implanted in the uterine wall. The decidua contains mother's stem cells. The amount of stem cells contained in the placenta is very abundant, and placenta stem cells well proliferate and can also differentiate into other cells.

Decidua- or amnion-derived stem cells isolated from the human term placenta according to the present invention are classified as autologous adult stem cells, which do not cause ethical problems, because they are derived from placenta tissue.

Multipotent stem cells are generally isolated and purified from placenta tissue through the following method. The present invention relates to mammalian placenta, and preferably human placenta. After expulsion from the uterus, the placenta is treated and cultured to produce multipotent stem cells, placenta stem cells and other biomaterials. Placenta stem cells are obtained from the placenta after expulsion from the uterus. In a preferred embodiment, the placenta is cultured in the presence of growth factors [(e.g., bFGF (basic Fibroblast Growth Factor) and EGF (Epidermal Growth Factor)].

In the present invention, placentas were collected from normal births and premature births in Guro Hospital, Korea University Medical Center, according to the Institutional Review Board guidebook of Korea University Medical Center, and multipotent stem cells were isolated and purified from the human placenta tissue through the following method.

Amnion and decidua were isolated from human placenta tissue, and washed with PBS. The washed amnion and decidua tissues were finely cut. The finely cut amnion and decidua tissues are moved to a 100-mm dish, and then chemically decomposed in a DMEM (Dulbecco's Modified Eagle Medium, Gibco) medium supplemented with collagenase (1 mg/ml) at 37 °C for 1 hour.

The chemically decomposed tissues were filtered through a 100µm mesh to remove non-decomposed tissues, and then centrifuged at 1200 rpm for 1-10 minutes. The supernatant was suctioned, and pellets remaining on the bottom were washed with PBS and then centrifuged at 1200 rpm for 1-10 minutes. Pellets remaining on the bottom were well suspended as single cells and then cultured in a bFGF-containing DMEM medium. At this time, mesodermal stem cells were attached to the bottom, and the other cells were suspended.

Such stem cells grew attached to plastic and showed a round-shaped or spindle-shaped morphology. After two days, cells unattached to the dish bottom were washed with PBS and cultured while replacing the medium at an interval of 2-3 days, thus obtaining a solution of amnion-derived and decidua-derived multipotent stem cells isolated from the human placenta.

The proliferation rate of the isolated placenta-derived multipotent stem cells was examined and, as a result, it could be seen that these cells showed a gradual increase in CPDL up to passage 12, suggesting that these cells had excellent proliferation rate.

The placenta stem cells according to the present invention form spheres in a SFM medium, and thus can be maintained in a undifferentiated state for a long period of time. One example of a SFM medium usable in the present invention may be MEBM (mammary epithelial basal medium) containing 10 µM, 1 x antibiotic antimycotic solution, 1 µg/ml hydrocortisone, 5 µg/ml insulin, 20 ng/ml EGF, 40 ng/ml FGF, B27, and β-mercaptoethanol.

The number and type of cells propagated may easily be monitored by measuring changes in morphology and cell surface markers using standard cell detection techniques such as flow cytometry, cell sorting, immunocytochemistry (e.g., staining with tissue specific or cell-marker specific antibodies) fluorescence activated cell sorting (FACS), magnetic activated cell sorting (MACS), by examination of the morphology of cells using light or confocal microscopy, or by measuring changes in gene expression using techniques well known in the art, such as PCR and gene expression profiling.

In a preferred embodiment, cells cultured in the placenta are sorted using techniques known in the art, for example, density gradient centrifugation, magnetic cell separation, flow cytometry and other cell separation methods.

Methods of obtaining multipotent stem cells expressing the desired surface antigens from the placenta-derived stem cell broth obtained above include a FACS method using a flow cytometer with sorting function (Int. Immunol., 10(3):275, 1998), a method using magnetic beads, and a panning method using an antibody specifically recognizing multipotent stem cells (J. Immunol., 141 (8):2797, 1998). Also, methods for obtaining multipotent stem cells from a large amount of culture broth include a method where antibodies specifically recognizing molecules expressed on the surface of cells (hereinafter, referred to as "surface antigens") are used alone or in combination as columns.

Flow cytometry sorting methods may include a water drop charge method and a cell capture method. In one embodiment, cell surface marker-specific antibodies or ligands are labeled with distinct fluorescent labels. Cells are processed through a cell sorter, allowing separation of cells based on their ability to bind to the antibodies used. FACS- sorted particles may be directly deposited into individual wells of 96-well or 384-well plates to facilitate separation and cloning.

In any of these methods, an antibody specifically recognizing an antigen on the cell surface is fluorescently labeled, the intensity of fluorescence emitted from an antibody bonded with the molecule expressed on the surface of the cell is converted to an electric signal whereby the amount of the antigen expressed can be quantified. It is also possible to separate cells expressing a plurality of surface antigens by combination of fluorescence types used therefor. Examples of fluorescences which can be used in this case include FITC (fluorescein isothiocyanate), PE (phycoerythrin), APC (allophycocyanin), TR (Texas Red), Cy 3, CyChrome, Red 613, Red 670, TRI-Color, Quantum Red, etc.

FACS methods using a flow cytometer include: a method where the above stem cell broth is collected, from which cells are isolated by, for example, centrifugation, and stained directly with antibodies; and a method where the cells are cultured and grown in a suitable medium and then stained with antibodies. The staining of cells is performed by mixing a primary antibody recognizing a surface antigen with a target cell sample and incubating the mixture on ice for 30 minutes to 1 hour. When the primary antibody is fluorescently labeled, the cells are isolated with a flow cytometer after washing. When the primary antibody is not fluorescently labeled, cells reacted with the primary antibody and a fluorescent labeled secondary antibody having binding activity to the primary antibody are mixed after washing, and incubated in ice water for 30 minutes to 1 hour. After washing, the cells stained with the primary and secondary antibodies are isolated with a flow cytometer.

### 3. Characteristics of placenta stem cells isolated from placenta

The stem cells isolated from the placenta are homogenous, and sterile. Furthermore, the stem cells are readily obtained in a form suitable for administration to humans, i.e., they are of pharmaceutical grade.

After long-term culture, cells can be characterized with CD-series of surface antigen markers, for example, CD29 (mononuclear cell marker), CD31 (endothelial cell and stem cell marker), CD34, CD44 (hematopoietic cell marker), CD90 (mononuclear stem cell marker), CD73 (T-cell and B-cell marker), CD105 (endothelial cell marker), and CD45 (hematopoietic cell marker), and can be applied to FACS analysis.

Preferred placenta stem cells obtained by the method of the present invention may be identified by the presence of the following cell surface markers: showing a positive immunological response to CD29, CD44, CD54, CD73, CD90 and CD105 and showing a negative immunological response to CD31, CD34, CD45 and HLA-DR. Such cell surface markers are routinely determined according to methods well known in the art, e.g. by flow cytometry, followed by washing and staining with an anti-cell surface marker antibody.

Also, the placenta stem cells according to the present invention can be identified using an Oct4 or SSEA4 marker that can be considered as an undifferentiated cell marker. Oct4 is well known as an undifferentiated marker for stem cells, and it is general to test Oct4 expression ability in order to identify stem cells in an undifferentiated state, as disclosed in Korean Patent Publication No. 10-2006-0067199, entitled "Monoclonal antibody specific to human embryonic stem cells", Korean Patent Publication No. 10-2006-0057738, entitled "Double-stranded RNA for inhibition of expression of Oct4 gene that maintains undifferentiated state of mammalian embryonic stem cells", and the like. Also, it is well known that SSEA4 (stage specific embryonic antigen 4) is present on the surface of human embryonic stem cells.

The expression of Oct4 and SSEA4 employs RT-PCR (reverase transcriptase-polymerase chain reaction). The method of RT-PCR is a technique known in the art. RT-PCR is a technique comprising synthesizing corresponding cDNA using RNA of a specific region as a template and carrying out PCR amplification using the cDNA, and consists of (1) preparing cDNA from RNA using reverse transcriptase, and (2) amplifying a specific region using the cDNA, and the step (2) is the same as a method of amplifying a specific gene region from genomic DNA. This method can be performed in a simpler manner compared to RNA analysis which has been possible through methods such as Northern blot hybridization, and it allows the base sequence of a gene to be determined. Thus, this method is greatly useful mainly in studying the base sequence and transcription level of mRNA.

The placenta stem cells according to the present invention show a positive response to the expression of Oct4 and SSEA4.

### 4. Differentiation of placenta stem cells

The stem cells obtained according to the inventive method has the ability to differentiate into mesoderm-, endoderm- and ectoderm-derived cells. These cells may be induced to differentiate along specific cell lineages, including fat cell differentiation, cartilage cell differentiation, osteoblast differentiation, hematopoietic cell differentiation, muscle cell differentiation, vascular cell differentiation, nerve cell differentiation, and liver cell differentiation. In a specific embodiment, the placenta stem cells obtained according to the inventive method are induced to differentiate for use in transplantation and *ex vivo* treatment protocols. In a specific embodiment, the placenta stem cells obtained according to the inventive method are induced to differentiate into a particular cell type and are genetically engineered to provide a therapeutic gene product.

In a specific embodiment, the placenta stem cells obtained according to the inventive method are incubated with a compound, in vitro, that induces them to differentiate, followed by direct transplantation of the differentiated cells into a subject. Accordingly, the present invention encompasses a method for differentiating human placenta stem cells using a standard culture medium. Thus, the present invention encompasses a method for differentiating the multipotent placenta stem cells of the present invention into muscle cells, nerve cells, stellate cells, osteogenic cells, cartilage cells, fat cells, and insulin-secreting pancreatic β-cells.
The differentiation of stem cells into a particular cell type can be measured according to any method known in the art, and the placenta stem cells can be induced to differentiate into a particular cell type using, for example, the following methods: (1) The placenta stem cells can be induced to differentiate into muscle cells by pretreating the stem cells with azacytidine for one day and then culturing the pretreated cells in SKBM medium (Cambrex, Co.); (2) the placenta stem cells can be differentiated into nerve cells by preculturing the stem cells in a medium containing BME (β-mercaptoethanol) and FBS (fetal bovine serum) and treating the precultured broth with DMSO (dimethyl sulfoxide) and BHA (butylated hydroxyanisole); (3) the placenta stem cells can be differentiated into osteogenic cells by mixing the stem cells with TCP (tricalcium phosphate), followed by heterotopic transplantation; (4) the placenta stem cells can be differentiated into fat cells by culturing the stem cells in an α-MEM containing dexamethasone, indomethacin, insulin and IBMX (3-isobutyl-1-methylxanthine); and (5) the placenta stem cells can be differentiated into insulin-releasing pancreatic β-cells by preculturing the placenta stem cells in DMEM/20% CBS medium supplemented with alkaline fibroblast growth factor and transforming growth factor β-1, and culturing the precultured placenta stem cells by adding the medium from culture of nestin-positive nerve unit cells into said precultured placenta stem cells in the concentration ratio of 50:50.

Determination that stem cells have differentiated into a particular cell type may be accomplished by methods well-known in the art, e.g., measuring changes in morphology and cell surface markers (e.g., staining cells with tissue-specific or cell-marker specific antibodies) using techniques such as flow cytometry or immunocytochemistry, by examination of the morphology of cells using light or confocal microscopy, or by measuring changes in gene expression using techniques well known in the art, such as PCR and gene-expression profiling.

### 5. Use of placenta stem cells and cells differentiated therefrom

The placenta stem cells according to the present invention can be used for a wide variety of therapeutic protocols in which the tissue or organ of the body is augmented, repaired or replaced by the engraftment, transplantation or infusion of a desired cell population, such as a stem cell or progenitor cell population. The placenta stem cells of the present invention can be used to replace or augment existing tissues, to grow new or altered tissues, or to bond the tissues with biological tissues or structures. Also, in therapeutic protocols in which embryonic stem cells are typically used, the embryonic stem cells can be replaced with the placenta stem cells of the present invention. For example, the placenta stem cells of the present invention and cells differentiated therefrom can be used in cell therapeutic agents for treating nervous diseases, such as Alzheimer's disease and Parkinson's diseases, muscular diseases, such as progressive muscular dystrophy and Lou Gehrig's disease, osteodiseases, such as osteoarthritis and osteoporosis, and diabetes, and in cell therapeutic agents for forming breast tissue.

In a preferred embodiment of the present invention, the placenta stem cells or other stem cells from the placenta may be used in autologous and allogenic transplants, including HLA-matched and HLA-mismatched hematopoietic transplantations. For example, the placenta stem cells of the present invention can be used in therapeutic transplantation protocols, e.g., to augment or replace stem or progenitor cells of the liver, pancreas, kidney, lung, nervous system, muscular system, bone, bone marrow, thymus, spleen, mucosal tissue, gonads, or hair.

The placenta stem cells of the present invention may be used instead of specific classes of progenitor cells (e.g., chondrocytes, hepatocytes, hematopoietic cells, pancreatic parenchymal cells, neuroblasts, muscle progenitor cells, etc.) in therapeutic or research protocols in which progenitor cells would typically be used.

The placenta stem cells of the present invention can be used for augmentation, repair or replacement of cartilage, tendon, or ligaments. For example, in certain embodiments, prostheses (e.g., hip prostheses) are coated with cartilage tissue constructs grown from the placenta stem cells of the present invention. In other embodiments, joints (e.g., knee) are reconstructed with cartilage tissue constructs grown from the placenta stem cells. Cartilage tissue constructs can also be employed in major reconstructive surgery for different types of joints (for protocols, see e.g., Resnick, D. et al, Diagnosis of Bone and Joint Disorders, 2d, 1988).

The placenta stem cells of the present invention can be used to repair damage of tissues and organs resulting from disease. In such an embodiment, a patient can be administered the placenta stem cells to regenerate or restore tissues or organs which have been damaged as a consequence of disease, e.g., enhance immune system following chemotherapy or radiation, repair heart tissue following myocardial infarction.

In addition, the placenta stem cells of the present invention may be formulated as injectable preparations (e.g., WO 96/39101, incorporated herein by reference in its entirety). In an alternative embodiment, the cells and tissues of the present invention may be formulated using polymerizable or cross-linking hydrogels as described in US 5,709,854; US 5,516,532; and US 5,654,381; each of which is incorporated by reference in their entirety.

### Examples

Hereinafter, the present invention will be described in more detail by examples. It is to be understood, however, that these examples are for illustrative purpose only and are not construed to limit the scope of the present invention.

### Example 1: Preparation and isolation of placenta tissues

The placentas were collected from normal births and premature births in Guro Hospital, Korea University Medical Center, according to the Institutional Review Board guidebook of Korea University Medical Center, and were used for researches. The placenta tissues were transferred to the laboratory in a state in which it was contained in physiological saline containing an antibiotic.

The placenta tissues transferred to the laboratory were washed with PBS to remove blood cells and various other tissues, or the tissues were treated with hemolysis buffer to remove blood cells, or each of amnion, chorion, decidua and placental bed tissues constituting the placenta was carefully isolated using forceps.

### Example 2: Isolation and culture of placenta-derived stem cells

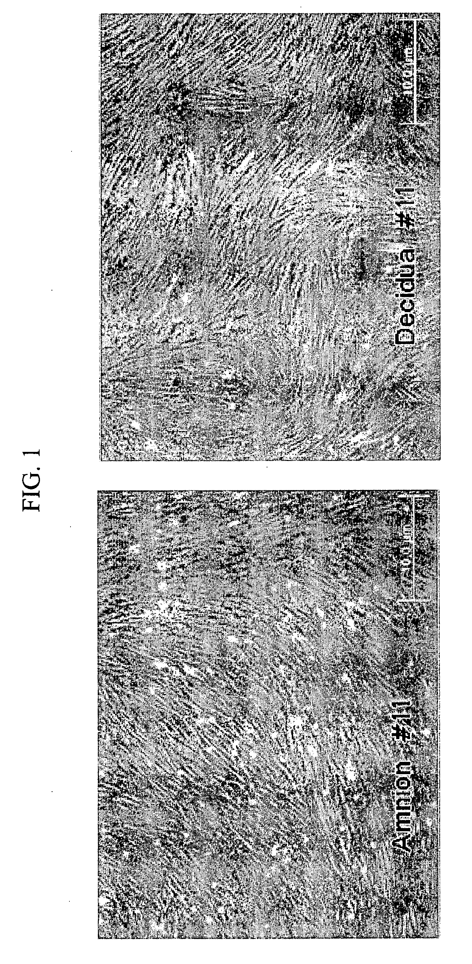

Each of the isolated tissues was placed on a 100-mm dish and finely cut with a sterilized scalpel to a size of 1-2 mm. Then, the cut tissue was placed in a collagenase-containing medium, was allowed to react in an incubator at 37°C for 1-4 hours, after which the tissues treated with collagenase were filtered through 100-mesh wire cloth. The cells thus isolated were placed on a 100-mm dish and cultured in a DMEM medium at 37°C in a condition of 5% CO₂. FIG. 1 is a microscopic photograph showing the morphology of amnion-derived and decidua-derived mesodermal stem cells.

### Comparative Example 1: Comparison of culture efficiency between placenta tissue-derived and fat tissue-derived stem cells

When stem cells isolated from 5 g of fat tissue were cultured, more than 10,000,000 cells could be obtained after second-passage culture, but in the case of about 3 g of placenta tissue, more than 10,000,000 cells could be obtained after first-passage culture. From such results, it could be seen that the case of culturing stem cells using placenta tissue was much more efficient than the existing case of culturing stem cells using fat tissue.

### Example 3: Examination of proliferation rate and sphere formation of placenta tissue-derived stem cells

The proliferation rate of the stem cells obtained according to the above method of proliferating the human placenta tissue-derived multipotent stem cells was examined. Placenta stem cells resulting from the placenta tissue samples of different human individuals were obtained through the isolation method described in Examples 1 to 3, and then seeded into a 75-flask at a density of 2x10⁵ cells.

CPDL is an index indicative of the proliferation rate of cells and expressed as the following equation.

CPDL = In(Nf/Ni)/ln2, wherein Ni: the initial number of seeded cells; and Nf: the final number of cells.

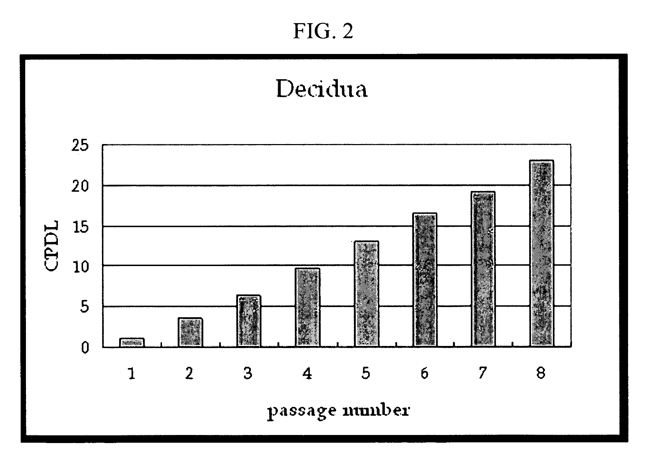

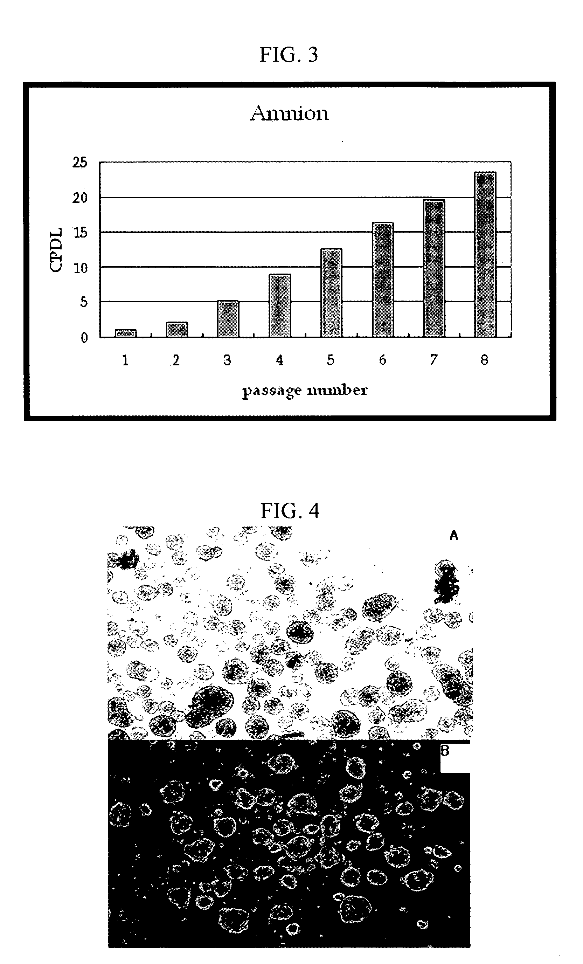

The CPDL of the amnion-derived stem cells and decidua-derived stem cells was observed according to passage number and, as a result, the cells showed a CPDL value of about 30 at passage 12 (see FIGs. 2 and 3). This CPDL value was similar to that of human fat tissue-derived stem cells (Lin et al., Stem Cells and Development, 14:92, 2005; Zuk et al., Tissue Eng., 7:211, 2001). These results suggest that the adult stem cells according to the present invention have very high proliferation rate.

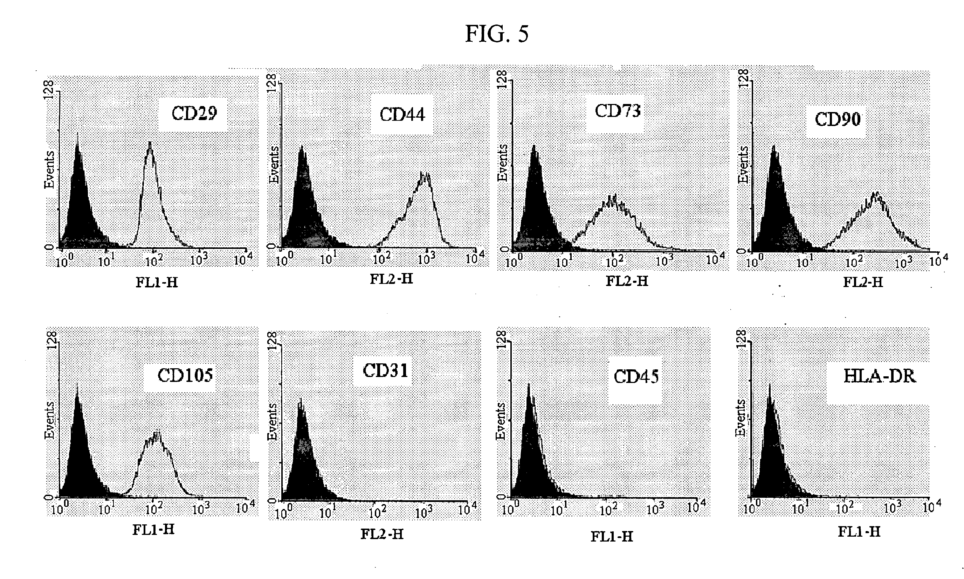

Meanwhile, the amnion-derived stem cells and decidua-derived stem cells according to the present invention were cultured in MEBM (mammary epithelial basal medium, containing 10 µM, 1 x antibiotic antimycotic solution, 1 µg/ml hydrocortisone, 5 µg/ml insulin, 20 ng/ml EGF, 40 ng/ml FGF, B27 and β-mercaptoethanol), one of SFM media, for 3 days and 7 days, respectively. As a result, as shown in FIG. 4, the placenta stem cells according to the present invention formed spheres in the SFM medium, suggesting that the cells would be maintained in an undifferentiated state for a long period of time.

### Example 4: Immunological characteristics of placenta-derived multipotent stem cells

The decidua-derived stem cells and amnion-derived stem cells obtained in Example 3 were washed with PBS and treated with trypsin. The treated cells were collected and centrifuged at 1000 rpm for 5 minutes. The supernatant was discarded and then washed with a mixture of 5% FBS and PBS, followed by centrifugation at 1000 rpm for 5 minutes. The supernatant was discarded, and the cells were suspended in PBS, and 1×10⁵ cells for each sample were dispensed into a well plate. An antibody (R-phycoerythrin-conjugated mouse anti-human monoclonal antibody) was placed into each well and incubated on ice for 40 minutes. After the incubation, the medium was centrifuged at 1000 rpm for 5 minutes. The supernatant was removed and the cells were washed with PBS and centrifuged at 1000 rpm for 5 minutes. Once again, the supernatant was removed, and the cells were washed with PBS and centrifuged at 1000 rpm for 5 minutes. After removing the supernatant, the cells were fixed with 1% paraformaldehyde and analyzed using a flow cytometer.

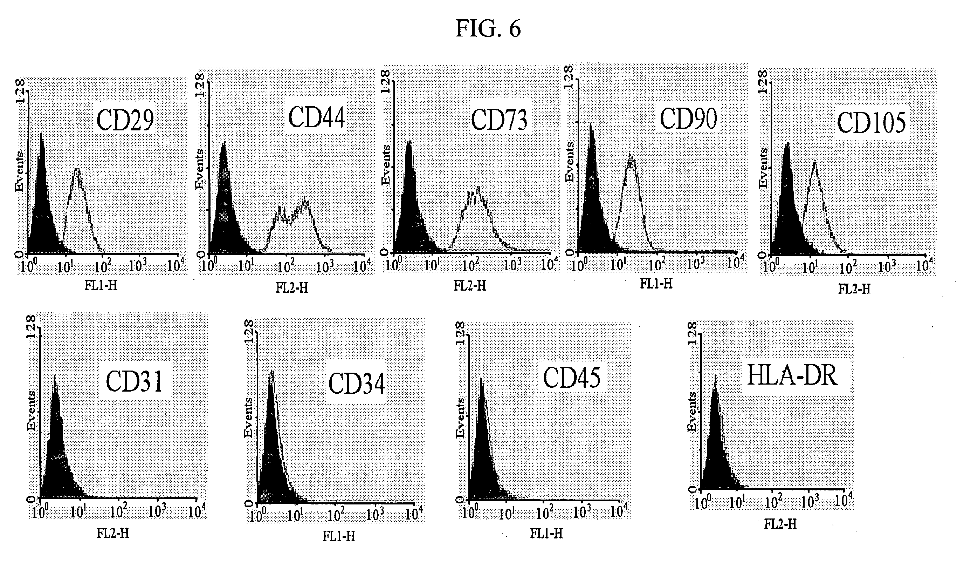

As a result, as shown in Table 1, FIGs. 5 and 6, it could be seen that the placenta-derived stem cells [decidua-derived stem cells (FIG. 5); amnion-derived stem cells (FIG. 6)] of the present invention all showed a positive immunological response to CD29, CD44, CD73, CD90 and CD105, and showed a negative immunological response to CD31, CD34, CD45 and HLA-DR.

**Table 1: Surface antigen analysis (FACS analysis) of placenta-derived stem cells**

| Antigen | AD-MSCs |
|---|---|
| CD29 | + |
| CD31 | - |
| CD44 | + |
| CD90 | + |
| CD105 | + |
| CD34 | - |
| CD45 | - |
| CD73 | + |
| CD34 | - |
| HLA-DR | - |

### Example 5: Analysis of Oct4 and SSEA expression of placenta-derived multipotent stem cells

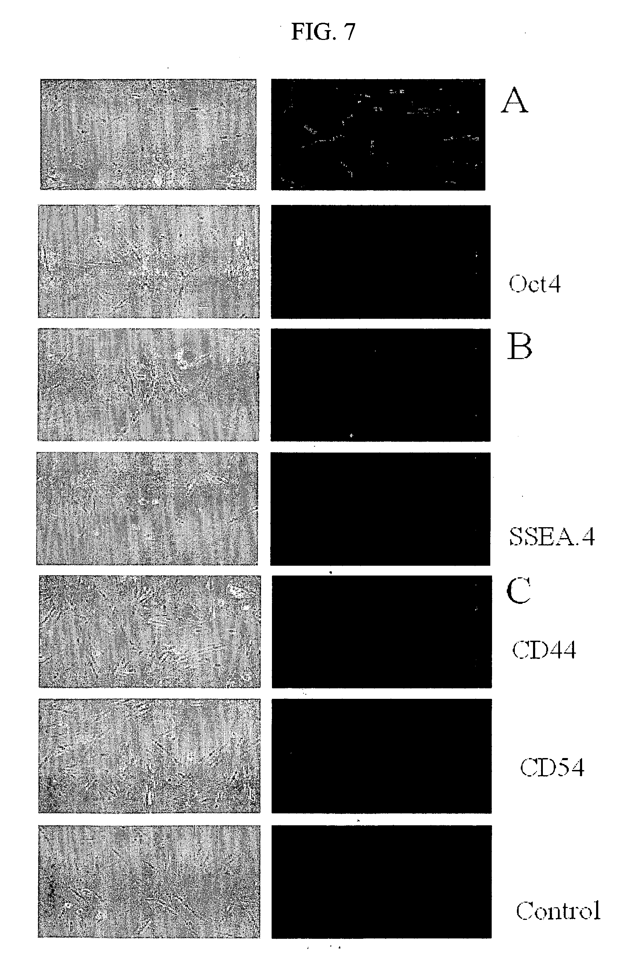

The placenta-derived stem cells obtained in Example 3 were washed three times with PBS, and immobilized with 4% paraformaldehyde-containing PBS for 30 min. After washing three times with PBS, the cells were permeabilized with 0.1 % Triton-X100-containing PBS for 10 min. After washing three times with PBS, the cells were allowed to react with blocking buffer (5% goat serum) at 4°C for one hour, and then allowed to react with primary antibody-containing blocking buffer overnight. After washing three times with PBS, the cells were allowed to react with a secondary antibody in a dark room for 1 hour. After washing three times with PBS, the cells were mounted. As a result, the stem cells according to the present invention showed a positive response to Oct4 and SSEA that are markers for human embryonic stem cells (see FIG. 7).

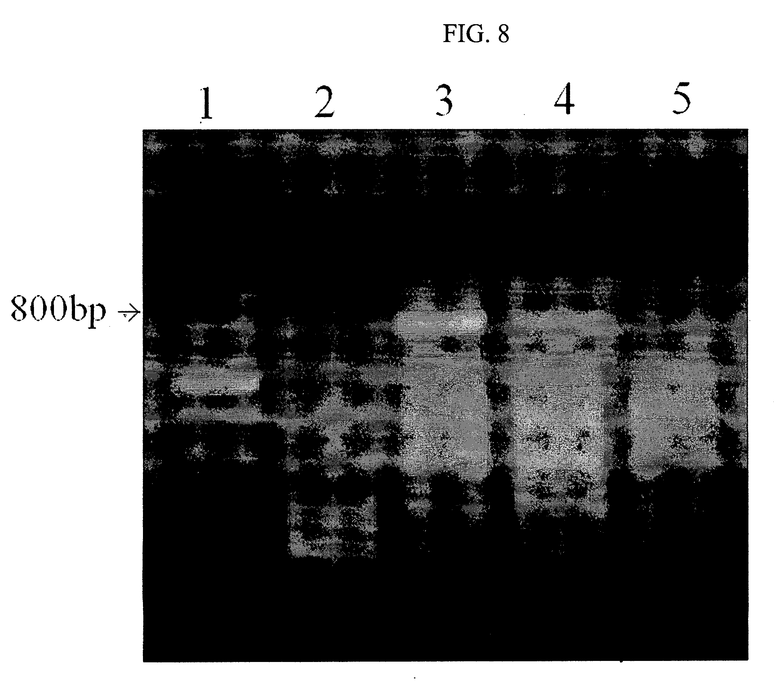

Also, the expression of Oct4 was analyzed using RT-PCR. The RT reaction was performed for 50 minutes at 37°C and 10 minutes at 70°C, and the PCR reaction was performed for 5 minutes at 95°C, and 40 cycles, each consisting of 30 sec at 95°C, 40 sec at 58°C and 1 min at 72°C, and then 10 minutes at 72°C. As a result, as shown in FIG. 8, it could be seen that the decidua-derived stem cells and the amnion-derived stem cells were expressed at 800 bp.

### Example 6: Differentiation of placenta tissue-derived multipotent stem cells into muscle cells

The placenta tissue-derived multipotent stem cells obtained in Example 3 were dispensed into a 10 ng/ml fibronectin-coated flask and then pretreated with 10 µM 5'-azacytidine for 24 hours. After the pretreatment, the cells were cultured in a muscle cell differentiation-inducing medium (SKBM medium; MM-3160; Cambrex, Co.) for 10 day, followed by immunostaining.

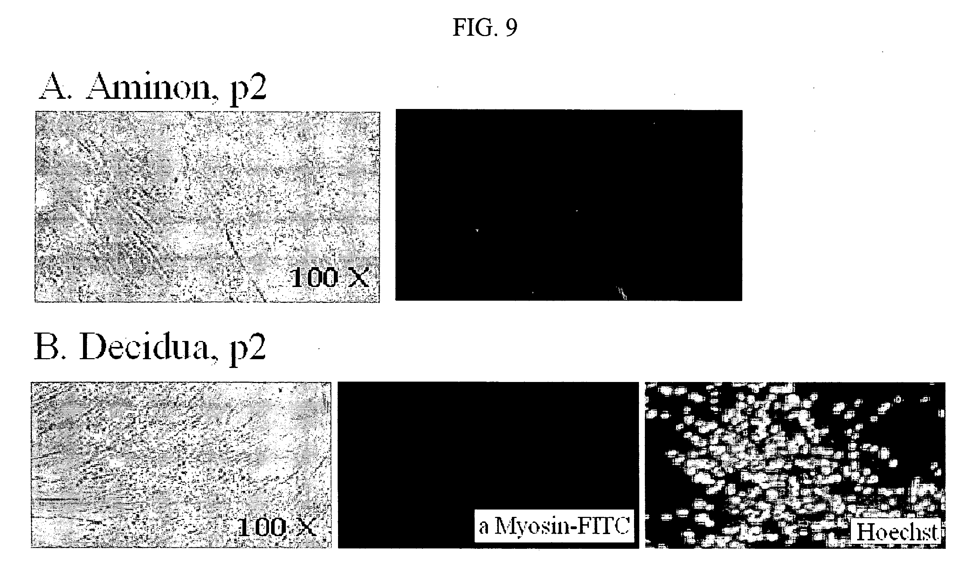

As a result, as shown in FIG. 9, the placenta tissue-derived multipotent stem cells according to the present invention showed a positive response to myosin that is a muscle cell-specific antegen. This result suggests that the human placenta tissue-derived multipotent stem cells according to the present invention were differentiated into muscle cells.

### Example 7: Differentiation of placenta tissue-derived multipotent stem cells into nerve cells

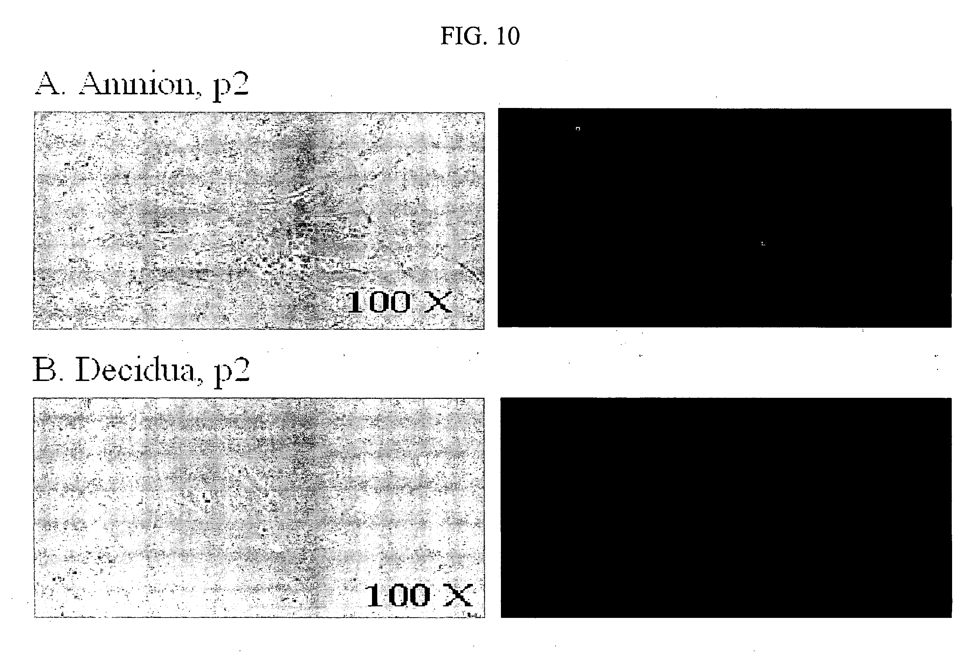

The placenta tissue-derived multipotent stem cells obtained in Example 3 were preincubated in a DMEM medium supplemented with 1 mM BME and 10% FBS, for 24 hours. After the preincubation, the stem cells were incubated in nerve cell differentiation-inducing medium (NM 3229: Cambrex, Co.), containing 1% DMSO and 100 µM BHA (butylated hydrxyanisole), for 9 days, so as to induce differentiation into nerve cells, followed by immunostaining. As a result, as shown in FIG. 10, the placenta tissue-derived multipotent stem cells according to the present invention showed positive responses to GFAP (glial fibrillary acidic protein). This result suggests that the human placenta tissue-derived multipotent stem cells according to the present invention were differentiated into nerve cells.

### Example 8: Differentiation of placenta tissue-derived multipotent stem cells into osteogenic cells

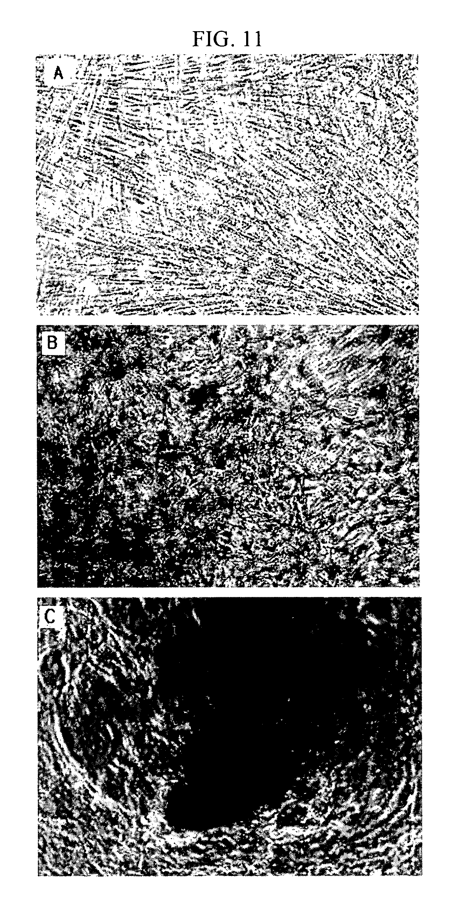

The placenta tissue-derived stem cells obtained in Example 3 were diluted in an osteogenic medium (containing 0.1 µmol/L dexamethasone, 0.05 mmol/L ascorbic acid-2-phosphate, and 10 mmol/L β-glycophsphate, 5-30% human serum or plasma), and then cultured in a flask in the presence of 5% CO₂ at 37°C while replacing the medium at an interval of 3-4 days, thus inducing differentiation into osteogenic cells. At 14 days after the start of the culture, the differentiation of the placenta tissue-derived stem cells into osteogenic cells was confirmed using Alizalin red S staining (see FIG. 11).

### Example 9: Differentiation of placenta tissue-derived multipotent stem cells into fat cells

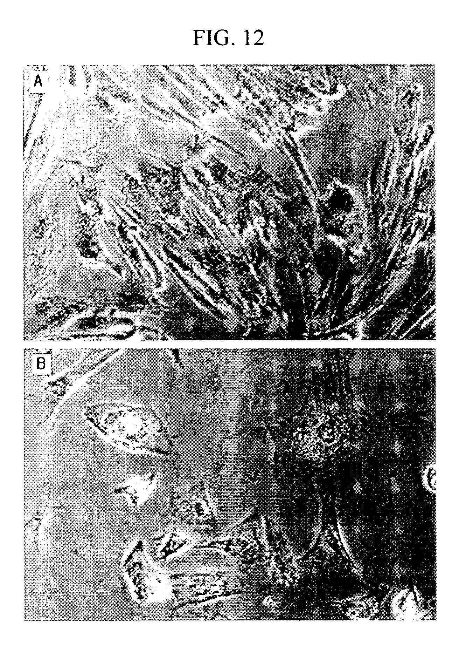

The placenta tissue-derived stem cells obtained in Example 3 were incubated in an α-MEM medium containing 5% FBS, 1 µM dexamethasone, 200 µM indomethacin, 10 µg/ml insulin and 0.5 mM IBMX (3-isobutyl-1-methylxanthine) for 2 weeks to induce differentiation into fat cells and then analyzed using an oil red O staining method. As a result, as shown in FIG. 12, it was observed that the placenta tissue-derived stem cells according to the present invention were differentiated into fat cells.

As described above in detail, the inventive method of producing placenta stem cells using placenta tissue can more efficiently produce the stem cells, compared to the existing methods of producing stem cells from other tissues. Also, the multipotent stem cells according to the present invention have the ability to differentiate into, for example, muscle cells, vascular endothelial cells, osteogenic cells, nerve cells, satellite cells, fat cells, cartilage-forming cells, osteogenic cells, or insuline-secreting pancreatic β-cells, and thus are effective for the treatment of liver cirrhosis, osteoporosis, osteoarthritis, nervous diseases, diabetes and the like, and are useful for the formation of breast tissue.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A method for producing placenta stem cells having the following characteristics, the method comprising culturing finely cut amnion, chorion, decidua or placenta tissue in a medium containing collagenase and bFGF and collecting the cultured cells:
(a) showing a positive immunological response to CD29, CD44, CD 54, CD73, CD90 and CD105, and showing a negative immunological response to CD31, CD34, CD45 and HLA-DR;
(b) showing a positive immunological response to Oct4 and SSEA4;
(c) growing attached to plastic, showing a round-shaped or spindle-shaped morphology, and forming spheres in an SFM medium so as to be able to be maintained in an undifferentiated state for a long period of time; and
(d) having the ability to differentiate into mesoderm-, endoderm- and ectoderm-derived cells.

2. Placenta stem cells produced by the method of claim 1, which have the following characteristics:
(a) showing a positive immunological response to CD29, CD44, CD73, CD90 and CD105, and showing a negative immunological response to CD31, CD34, CD45 and HLA-DR;
(b) showing a positive immunological response to Oct4 and SSEA4;
(c) growing attached to plastic, showing a round-shaped or spindle-shaped morphology, and forming spheres in an SFM medium so as to be able to be maintained in an undifferentiated state for a long period of time; and
(d) having the ability to differentiate into mesoderm-, endoderm- and ectoderm-derived cells.

3. The placenta stem cells according to claim 2, wherein the mesoderm-derived cells are selected from the group consisting of: muscle cells, osteogenic cells, cartilage cells, nerve cells, astrocytes, fat cells, insulin-releasing pancreatic β-cells and blood vessel endothelial cells.

4. A method for differentiating the placenta stem cells into muscle cells, the method comprising: pretreating the placenta stem cells of claim 2 with azacytidine; and then culturing said pretreated placenta stem cells in SKBM medium.

5. A cellular therapeutic agent for treating muscle disease, which contains the muscle cells differentiated by the method of claim 4, as an active ingredient.

6. A method for differentiating the placenta stem cells into nerve cells, the method comprising:
(a) preculturing the placenta stem cells of claim 2 in DMEM nedium containing BME and FBS; and
(b) treating the precultured broth with DMSO and BHA so as to induce differentiation into nerve cells.

7. A cellular therapeutic agent for treating nerve disease containing the nerve cells differentiated by the method of claim 6, as active ingredients.

8. A method for differentiating the placenta stem cells into osteogenic cells, the method comprising: mixing the placenta stem cells of claim 2 with tricalcium phosphate (TCP); and then isotransplanting the mixture.

9. A cellular therapeutic agent for treating osteoporosis, which contains the osteogenic cells differentiated by the method of claim 8, as an active ingredient.

10. A method for differentiating the placenta stem cells into fat cells, the method comprising: culturing the placenta stem cells of claim 2 in α-MEM medium containing dexamethasone, indomethacin, insulin and IBMX.

11. A cellular therapeutic agent for forming breast tissue, which contains the fat cells differentiated by the method of claim 10, as an active ingredient.

12. A method for differentiating the placenta stem cells into cartilage cells, the method comprising culturing the placenta stem cells of claim 2 in DMEM medium containing MSCGM and TFG-β3.

13. A cellular therapeutic agent for treating osteoarthritis, which contains the cartilage cells differentiated by the method of claim 12, as an active ingredient.

14. A method for differentiating the placenta stem cells into insulin-releasing pancreatic β-cells, the method comprising the steps of:
(a) preculturing the placenta stem cells of claim 2 in DMEM/20% CBS medium supplemented with alkaline fibroblast growth factor and transforming growth factor β-1; and
(b) culturing the precultured placenta stem cells by adding the medium from the culture of nestin-positive nerve unit cells into said precultured placenta stem cells.

15. A cellular therapeutic agent for treating diabetes, which contains insulin-releasing pancreatic β-cells differentiated by the method of claim 14, as an active ingredient.

16. A cellular therapeutic agent for treating muscle disease containing the placenta stem cells of claim 2, which have the ability of differentiation into muscle cells, as an active ingredient.

17. A cellular therapeutic agent for treating nerve disease containing the placenta stem cells of claim 2, which have the ability of differentiation into nerve cells, as an active ingredient.

18. A cellular therapeutic agent for treating osteoarthritis containing the placenta stem cells of claim 2, which have the ability of differentiation into cartilage cells, as an active ingredient.

19. A cellular therapeutic agent for treating bone deficiency containing the placenta stem cells of claim 2, which have the ability of differentiation into osteogenic cells, as an active ingredient.

20. A cellular therapeutic agent for forming breast tissue containing the placenta stem cells of claim 2, which have the ability of differentiation into fat cells, as an active ingredient.

21. A cellular therapeutic agent for treating diabetes containing the placenta stem cells of claim 2, which have the ability of differentiation into insulin-releasing pancreatic β-cells, as an active ingredient.
